# EUROPEAN PATENT SPECIFICATION

(11) **EP 2 379 081 B1**
(45) Date of publication and mention of the grant of the patent: **20.03.2013**
(21) Application number: 09802112.4
(22) Date of filing: 21.12.2009
(51) Int. Cl.: A61K 31/56, A61P 11/12

(54) **TREATMENT OF DIARRHOEA**
BEHANDLUNG VON DIARRHÖ
TRAITEMENT DE LA DIARRHÉE

(30) Priority: 19.12.2008 US 139459 P; 19.12.2008 EP 08172412
(43) Date of publication of application: 26.10.2011
(73) Proprietor: Royal College of Surgeons in Ireland, Dublin 2 (IE)
(72) Inventor: KEELY, Stephen, J., Dublin, 2 (IE); KEATING, Niamh, Dublin 2 (IE)
(74) Representative: Purdy, Hugh Barry
(86) International application number: PCT/EP2009/009198
(87) International publication number: WO 2010/069604

(56) References cited:
- EP-A- 1 886 685
- US-A1- 2006 069 070
- US-A1- 2007 015 796
- DOGGRELL S A: "Farnesoid X receptor agonism - A new approach to the treatment of cholesterol gallstone disease" EXPERT OPINION ON INVESTIGATIONAL DRUGS 200504 GB, vol. 14, no. 4, April 2005 (2005-04), pages 535-538, XP002516640 ISSN: 1354-3784

## Description

### Technical Field

The invention relates to a method for the treatment or prevention of the specific clinical manifestation of dysregulated fluid transport, namely diarrhoea.

### Background to the Invention

Epithelial cells line the surface of the intestinal tract and are responsible for regulating the movement of fluid to and from the body. Fluid movement is driven by the active transport of ions and the establishment of osmotic gradients and in the intestine, it is the secretion of Cl⁻ ions that is the primary driving force for fluid secretion. Cl⁻ ion secretion is promoted by immune cell mediators and neurotransmitters that elevate levels of intracellular 2^{nd} messengers, most notably, Ca²⁺ and cAMP. Many disease conditions exist (eg. inflammatory bowel disease, infectious disease, celiac disease, irritable bowel syndrome, bile acid malabsorption) that are characterised by dysregulated intestinal epithelial and fluid transport with the clinical manifestation of diarrhoea. However, to date, there is still a lack of therapeutic options for directly targeting epithelial transport processes in treatment of such condition.

It is an object of the invention to overcome at least one of the above problems.

### Statements of Invention

The invention is based on the surprising finding that agonists of the farnesoid X receptor (FXR), especially synthetic selective and potent agonists, significantly inhibit the ability of intestinal epithelial cells to evoke Cl⁻ secretory responses to agonists that act through either the Ca²⁺ or cAMP-dependant signalling pathways. As Cl⁻ ion secretion is the primary driving force for fluid secretion in the intestine, FXR activation inhibits fluid secretion into the intestine. Thus, FXR agonists are useful agents in the prevention or treatment of diarrhoea, as evidenced in the in-vitro and in-vivo data presented herein. As such, the present invention is directed to a symptomatic treatment, and typically not a causal treatment, and may be applied in the treatment of people who have different diseases or conditions, but specifically for the treatment of the specific diarrhoeal symptoms of the disease or condition.

In a preferred embodiment of the invention, the FXR agonists for used in the methods or compositions of the invention are selective and/or potent FXR agonists. The literature describes many such FXR ligands, generally synthetic ligands, which are synthetic and potent agonists of the FXR. In fact, the terms "potent selective" or "potent specific" as applied to synthetic FXR ligands would be recognised by a person skilled in this field as being synthetic molecules which bind specifically to the FXR ligand without any significant binding to most or all other nuclear receptors, and which have a potency or activity greater than the natural bile acid CDCA when measured in a conventional in-vitro assay (an example of a suitable assay for activity is provided in WO2008/02573). Typically, potent FXR agonists for use in the methods or compositions the invention would have an activity or potency as determined using the Cell Free Assay of Pelliciari (as described in WO2008/02573) in the nanomolar range, for example between 1 and 1000 nM, preferably between 1 and 500nM, preferably between 1 and 200nM, preferably between 1 and 100nM. Suitably, potent FXR agonists for use in the methods or compositions of the invention would have a potency or activity of between 100 and 1000 relative to the potency/activity of CDCA when measured using the Cell Free Assay of Pelliciari. Examples of literature describing synthetic FXR agonists, including synthetic potent and/or selective FXR ligands, and described below.

Accordingly, the invention broadly relates to a method for the treatment or prevention of diarrhoea (or diarrhoeal disease) in an individual comprising a step of administering 3 therapeutically effective amount of an FXR agonist to the individual.

The invention also relates to a method for the treatment or prevention of diarrhoea (or diarrhoeal disease) in an individual comprising a step of administering a therapeutically effective amount of a potent and/or selective FXR agonist, preferably a synthetic potent and selective FXR agonist, to the individual.

The invention also relates to a method for the treatment or prevention of dysregulated fluid transport into the intestine in an individual comprising a step of administering a therapeutically effective amount of an FXR agonist, suitably a potent and/or selective FXR agonist, preferably a synthetic potent and selective FXR agonist, to the individual.

The invention also relates to a method for the treatment or prevention of a disease, condition, or pathology characterised by dysregulated fluid transport in an individual comprising a step of administering a therapeutically effective amount of an FXR agonist, suitably a potent and/or selective FXR agonist, preferably a synthetic potent and selective FXR agonist, to the individual.

The invention also relates to the use of FXR agonists, especially potent FXR ligands, preferably synthetic potent and selective FXR agonists, ideally synthetic selective FXR agonists, in the treatment or prevention of diarrhoea or other symptoms of dysregulated fluid transport.

The invention also relates two the use of an FXR agonist, especially potent FXR agonists, preferably potent and selective FXR agonists, ideally synthetic selective FXR agonists, in the manufacture of a medicament for the treatment of a symptom of dysregulated fluid transport, typically diarrhoea or diarrhoeal disease.

The invention also relates to a method of treating or preventing dysregulated fluid transport (or the clinical manifestation of dysregulated fluid transport, namely diarrhoea) in an individual in which the dysregulated fluid transport is caused by a microbial infection comprising a step of treating the individual with a combination therapy comprising an anti-microbial agent sufficient to inhibit or retard the infection and a therapeutically effective amount of an FXR agonist, especially a potent FXR agonist, ideally a synthetic potent and/or selective FXR agonist.

The invention also relates to a pharmaceutical composition comprising an anti-microbial agent, an FXR agonist, and a suitable pharmaceutical carrier. Preferably, the FXR agonist is a potent FXR agonists, more preferably a potent synthetic FXR ligand, generally a selective FXR agonist, ideally a synthetic potent and selective FXR agonist.

Ideally, the FXR agonists employed in the methods and composition of the invention are potent agonists of the FXR receptor, preferably potent and selective FXR agonists, and ideally synthetic, potent and selective FXR agonists.

### Definitions

In this specification, the term "farnesoid X receptor (FXR)" should be taken to mean the orphan nuclear receptor initially identified from a rat liver cDNA library (BM. Forman, et al., Cell 81:687-693 (1995)) that is most closely related to the insect ecdysone receptor. It is a member of the nuclear receptor family of ligand-activated transcription factors that includes receptors for the steroid, retinoid, and thyroid hormones. It is most abundantly expressed in the liver, kidney, adrenal, and intestine.

In this specification, the term "FXR agonist" should be taken to mean compounds that function by targeting and selectively binding the farnesoid X receptor (FXR) and which activate FXR by at least 40% above background in the assay described in Maloney et al. (J. Med. Chem., 43:2971-2974). Examples of FXR agonists will be well known to the skilled person from the literature, and include the class of substituted isoxazole compounds described in US2008/0096921A1 (the complete contents of which are included herein by reference), including the compounds of generic formulae (I), or pharmaceutical salts thereof, recited on Page 2, Paragraphs 0010 to 0024, and the specific compounds recited in Paragraphs 0125 to 0163, and pharmaceutical salts thereof. Other FXR agonists are described in, for example, WO00/76523, WO00/40965, WO03/015771, WO03/015777 WO03/016280 WO03/016288, WO03/030612 US6, 984, 560, WO2008/002573, US2007/0015796, and US2008/0038435.

In a preferred embodiment of the invention, the FXR agonist is GW4064 described in US US2007/0015796.

In this specification, the term "selective FXR agonist" should be taken to mean an FXR agonist that exhibits no significant cross-reactivity to one or more, ideally substantially all (for example 10, 11, 12, 13, 14 or 15), of a panel of nuclear receptors consisting of LXRα, LXRβ, PPARα, PPARγ, PPARδ, RXRα, RARγ, VDR, SXR, ERα, ERβ, GR, AR, MR and PR. Methods of determining significant cross-reactivity are described in J. Med. Chem, 2009,52, 904-907 (supplementary data).

The term "potent" as applied to an FXR agonist should be understood to mean having an activity or potency greater that the activity/potentcy of CDCA when measured in the Cell Free Assay of Pelliciari, typically a relative activity/potency of 10 to 1000, ideally 100 to 1000 (i.e. 10 to 1000 times more potent than CDCA when measured using the Pelliciari assay). Thus, the potent FXR agonists useful for the methods and compositions of the present invention generally have a nM potency/activity, typically as determined using the Pelliciari assay, as opposed to the µM potency/activity of CDCA and other natural bile acid FXR ligands.

Examples of selective and/or potent FXR agonists are described in the following publications:

WO00/37077 (see compounds of general Formula I, Claim 1, and II of this Patent Application, especially compound GW4064 of Formula II the synthesis of which is described in Example 5, and potent derivatives thereof as described therein);
EP1392714 (6-ethyl-chenodeoxycholic acids, especially 3α, 7α-dihydroxy 7α-dihydroxy-6α-ethyl-5β-cholan-24-oic acid, also referred to as INT-747, and potent derivatives thereof as described therein);
EP1568706 (6-ethyl-ursodeoxycholic acids, and potent derivatives thereof as described therein);
WO2008/02573 (especially compounds IN-747, INT-1103 and UPF-987, and potent derivatives thereof as described therein);
J Med Chem. 2009 Feb 26;52(4):904-7. [Discovery of XL335 (WAY-362450), a highly potent, selective, and orally active agonist of the farnesoid X receptor (FXR)], and potent derivatives of XL355 as described therein; and Proc Natl Acad Sci U S A. 2008 Apr 8;105(14):5337-42. Epub 2008 Apr 7 [Identification of a potent synthetic FXR agonist with an unexpected mode of binding and activation], and EP0465142 (Merck) (especially compound 17-(4-hydroxybenzoyl)androsta-3,5-diene-3-carboxylic acid, known as MFA-1, and potent derivatives thereof as described therein). MFA-1 is a potent and selective FXR ligand having similar activity and structure to INT-747.

The term "potent derivatives thereof" as applied to the compounds GW-4064, INT-747, XL355, and MFA-1 means derivatives of these compounds described in the above-referenced publications having at least a nM potency, preferably a potency of between 10nM and 500nM, and ideally a potency of from 100 to 1000 times greater than the potency of CDCA as determined using the Cell Free Assay of Felliciari.

In this specification, the term "treatment or prevention" should be taken to mean both prophylactic and symptomatic treatment of an individual, including an individual who already exhibits the symptoms of dysregulated fluid transport, or an individual who does not yet have the symptoms of dysregulated fluid transport but who has been diagnosed with a condition characterised by dysregulated fluid transport. Typically, the term should be taken to include treatments which are effective in removing, inhibiting or ameliorating the symptoms. Ideally, the invention is directed to methods and composition for the symptomatic treatment of diarrhoea.

In the specification, the term "individual" should be taken to mean a human, however it should also include mammals for which the therapy of the invention is practicable.

In this specification, the term "therapeutically effective amount" should be taken to mean an amount of FXR agonist which results in a clinically significant reduction or prevention of the symptoms of diarrhoea. Suitably, the FXR agonist is administered at a dose of between 1 microgram and 50 milligrams daily, preferably between 1 milligram and 40 milligrams daily, more preferably between 5 milligrams and 40 milligrams daily.

In this specification, the term "diarrhea" or "diarrhoeal disease" should be understood to mean one, a plurality, or all of the diarrhoeal subtypes, including those selected from the group consisting of diarrhoea associated with inflammatory diseases (eg. Ulcerative colitis, Crohn's disease), Infectious diarrhoeas (eg. E.Coli, Salmonella, Clostridium difficile, cholera, campylobacter, rotoviruses etc), Irritable Bowel Syndrome (specifically, the IBS-D subtype), Drug-induced diarrhoeas (eg: chemotherapy-induced diarrhea, Bile acid-induced diarrhoea (eg: Short bowel syndrome, cholecystectomy etc), Diabetic diarrhea (can occur for a number of reasons including enteropathy or drug use), Allergic diarrhea (diarrhoea that occurs when "normal" food components are seen as antigenic), diarrhea associated with Coeliac disease (gluten hypersensitivity), and diarrhea associated with Carcinoid syndrome (tumour of enterochromaffin cells in the intestine leading to elevated serotonin). Thus, while the method of the invention may be employed to address a specific symptom of one or more of the above-referenced conditions, it is not directed to being a causal treatment of the diseases.

In this specification, the term "disease or condition or pathology characterized by dysregulated fluid transport" should be taken to mean inflammatory bowel disease, infectious disease, celiac disease, irritable bowel syndrome, any diseases or conditions associated with bile acid malabsorption, gastrointestinal infections, and other diseases or conditions that result in the clinical manifestation of diarrhea.

In this specification, the term "administering" should be taken to include any form of delivery that is capable of delivering the FXR agonist to colonic epithelial cells, including intravenous delivery, oral delivery, intramuscular delivery, intrathecal delivery, and inhaled delivery. Methods for achieving these means of delivery will be well known to those skilled in the art of drug delivery. For example administration of FXR agonists to animals is described in the following publications:
Hu et al. J. Biol. Chem., Vol. 281, Issue 52, 39831-39838, December 29, 2006; GW 4064 administered to rats by oral gavage (1 - 100 mg/kg)
Cariou et al., J. Biol. Chem., Vol. 281, Issue 16, 11039-11049, April 21, 2006 Administered to mice intraperitoneally (in corn oil) for 10 days (30 mg/kg)
Zhang et al., PNAS January 24, 2006 vol. 103 no. 4 1006-1011. GW 4064 administered to mice by oral gavage (30 mg/kg in 2-hydroxypropyl-β-cyclodextrin as vehicle), twice daily for 4 -11 days
Frankenberg et al., Am J Physiol Gastrointest Liver Physiol 290: G912-G922, 2006. GW 4064 Administered to mice by oral gavage (100 mg/kg in 1% Tween 80, 1% methylcellulose as vehicle) for 5 days
Lee et al., Journal of Lipid Research, Vol. 47, 201-214, January 2006 Mice were gavaged vehicle (2-hydroxypropyl-β-cyclodextrin) or GW4064 at 30 mg/kg twice a day for 4 days
Fiorucci et al., JPET 313:604-612, 2005. 6-ethyl-CDCA was administered intraperitoneally to rats (1 - 10 mg/kg) for 1 - 5 days
Stayrook et al., Endocrinology Vol. 146, No. 3 984-991 C57BL6 mice (7-9 wk of age) were treated with GW4064 (50 mg/kg twice daily, ip) or 5% acacia vehicle (four animals per group) for 7 days.
Liu et al, J Clin Invest. 2003 December 1; 112(11): 1678-1687. Rats were treated intraperitoneally with 5 ml/kg corn oil as vehicle or 30 mg/kg GW4064 in corn oil.

In one embodiment, the FXR agonist is formulated for oral administration and contains between 1 and 50mg of active. Inactive ingredients include lactose monohydrate, microcrystalline cellulose, pregelatinized starch, croscarmellose sodium, magnesium stearate, hypromellose, polyethylene glycol, polysorbate 80, and titanium dioxide.

Alternatively, the inactive ingredients may include colloidal silicon dioxide, crospovidone, hypromellose, lactose monohydrate, magnesium stearate, polyethylene glycol, polysorbate 80, povidone, pregelatinized starch, and titanium dioxide.

In this specification, the term "microbial infection" should be taken to mean a bacterial or viral infection of an individual which is characterised by causing dysregulated fluid transport into the intestine of the individual, generally causing the symptom of diarrhoea in the host. In most cases, such microbial infections are viral or bacteriological of the intestine caused by food-borne or air-borne microbes.

In this specification, the term "combination therapy" should be taken to mean that two pharmaceutically-active agents are co-administered to an individual. The agents may be in the form of a single formulation, be it a solid or liquid form, or may be provided as physically separate drugs that are co-administered to the individual.

In this specification, the term "anti-microbial" agent should be taken to mean a compound or drug that inhibits or retards the growth and/or multiplication of a microbe, and would include both anti-bacterial and/or anti-viral compounds, drugs or formulations.

In this specification, the term "pharmaceutical composition" should be taken to mean compositions comprising a therapeutically effective amount of an FXR agonist, and a pharmaceutically acceptable carrier or diluent. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the FXR agonist is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like.

The composition, if desired, can also contain minor amounts of wetting or emulsifying agents, or pH buffering agents. These compositions can take the form of solutions, suspensions, emulsion, tablets, pills, capsules, powders, sustained-release formulations and the like.

The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to, ease pain at the, site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

### Brief Description of the Figures

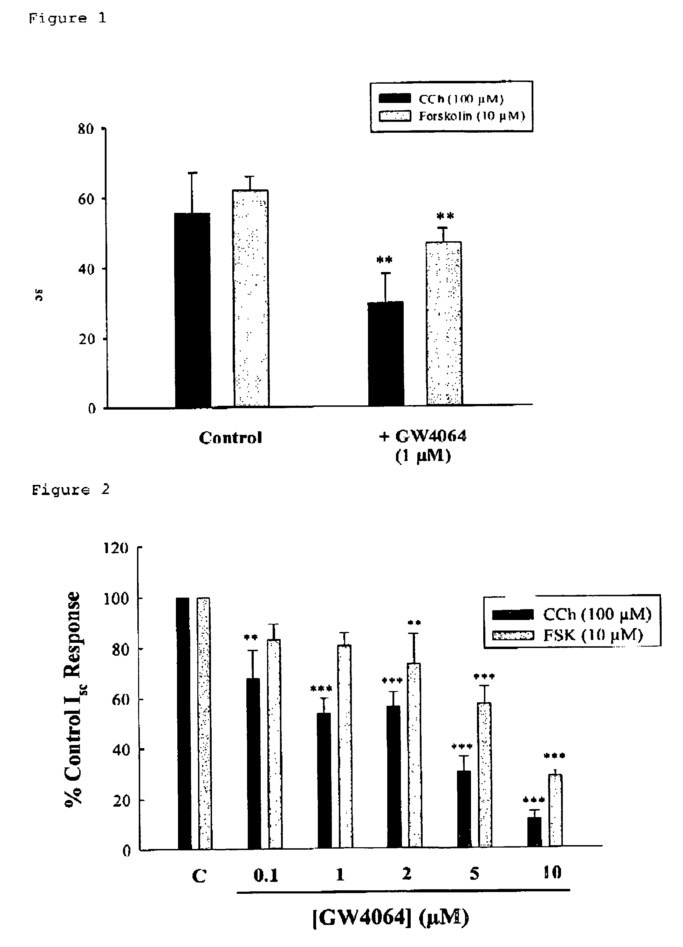

Figure 1: the effects of potent selective FXR agonist, GW 4064 (1 µM) on short circuit current (I_{sc}) responses to the Ca²⁺- and cAMP-dependent secretagogues, carbachol (CCh) and forskolin, respectively. Cells were pretreated with GW4064 for 24 hrs after which they were mounted in Ussing chambers and Cl⁻ secretory responses to CCh and forskolin were measured as changes in I_{sc}. GW 4064 was found to significantly reduce responses to both classes of secretagogue.
Figure 2: The concentration-dependence of the potent and selective FXR agonist, GW4064, in exerting its antisecretory actions.

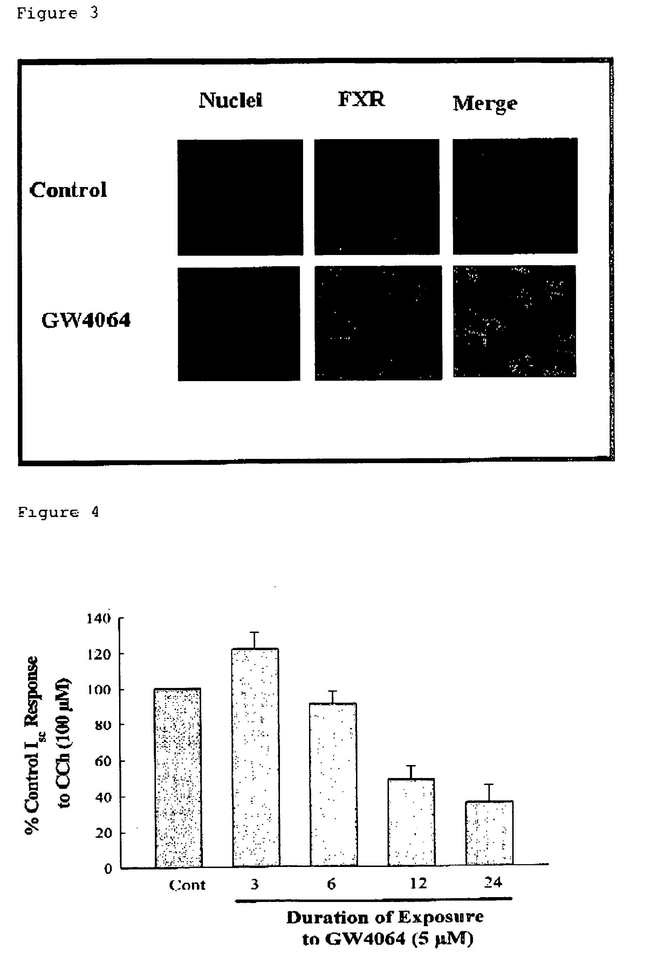

Figure 3: GW4064 stimulates FXR translocation to the nucleus. Subcellular localization of FXR (green) following treatment with the FXR agonist, GW4064 was investigated by immunocytochemistry and confocal microscopy. GW4064 (5 □M, 24hr) promoted the translocation of FXR from the cytoplasm to the nucleus (blue).
Figure 4: FXR activation attenuates Cl⁻ secretion in a time-dependent fashion: Monolayers of T₈₄ cells were pretreated with GW4064 (5 µM) for various periods of time. Cells were then washed and Cl⁻ secretory responses to CCh (100 µM) were measured in Ussing chambers. Data are expressed as % control I_{sc} responses to CCh (n = 2 - 3).

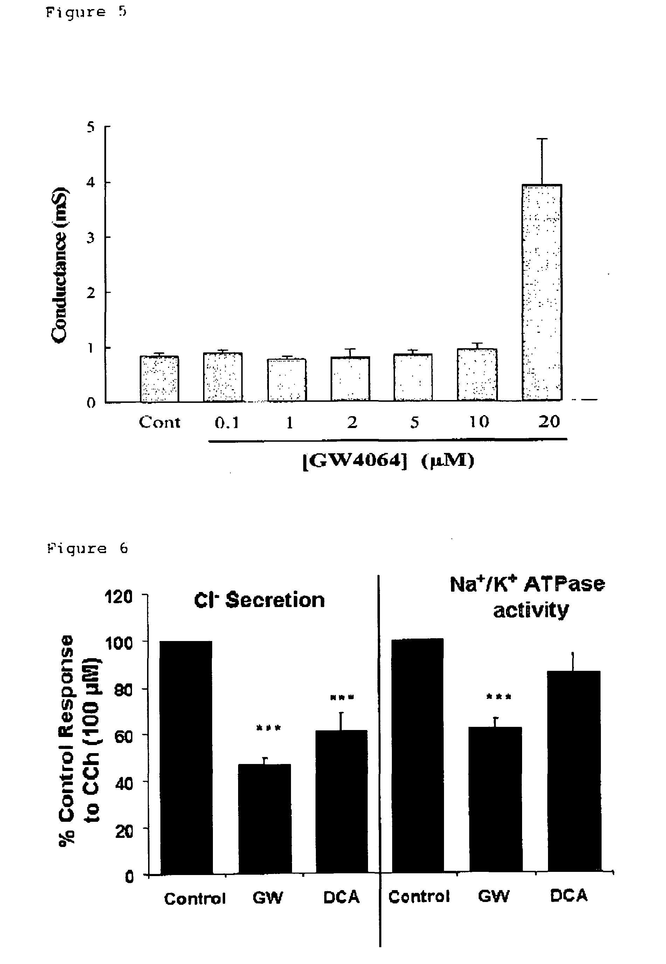

Figure 5: Effects of FXR activation on T₈₄ cell monolayer conductance: Monolayers of T₈₄ cells were treated with GW4064 at various concentrations for 24 hrs. Monolayers were then mounted in Ussing chambers and transepithelial conductance measurements were made as a measure of cell viability (n = 4 - 13). Only at concentrations > 10 µM did GW 4064 exert toxic effects on T₈₄ cells.
Figure 6: FXR activation inhibits Na⁺/K⁺ ATPase activity in colonic epithelial cells: Monolayers of T₈₄ cells were treated with GW4064 (5 µM) or deoxycholic acid (DCA; 50 µM) for 24 hrs and then mounted in Ussing chambers for measurements of Cl⁻ secretion (left panel; n = 3) or Na⁺/K⁺ ATPase activity (right panel; n = 4). Although both DCA and GW4064 inhibited CCh-induced Cl⁻ secretory responses, only GW 4064 inhibited Na⁺/K⁺ ATPase activity. *** p < 0.001.

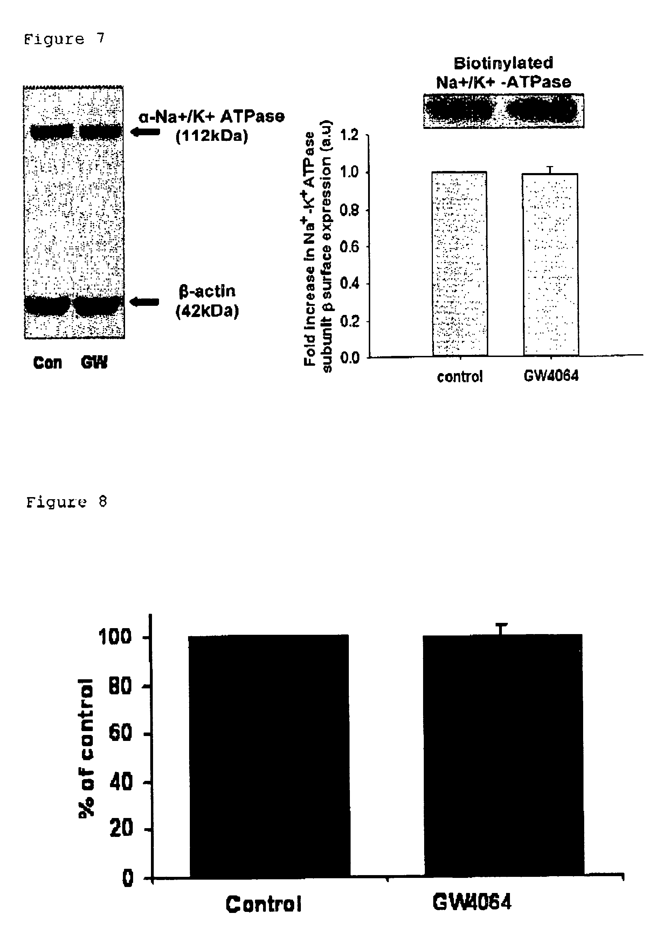

Figure 7: FXR activation does not alter abundance or surface expression of the Na⁺/K⁺ ATPase pump in T₈₄ cells: Monolayers of T₈₄ cells were treated with GW4064 (5 µM) for 24 hrs and then cellular abundance of the catalytic □ subunit was measured by western blotting (left panel; representative of 3 experiments) or its surface expression was measured by biotinylation (right panel; n = 4).
Figure 8: FXR activation does not alter cellular ATP levels in T₈₄ cells: Monolayers of T₈₄ cells were treated with GW4064 (5 µM) for 24 hrs and then cellular abundance ATP was measured using a commercially available assay kit panel. Data are expressed as % ATP levels in control cells (n = 5).

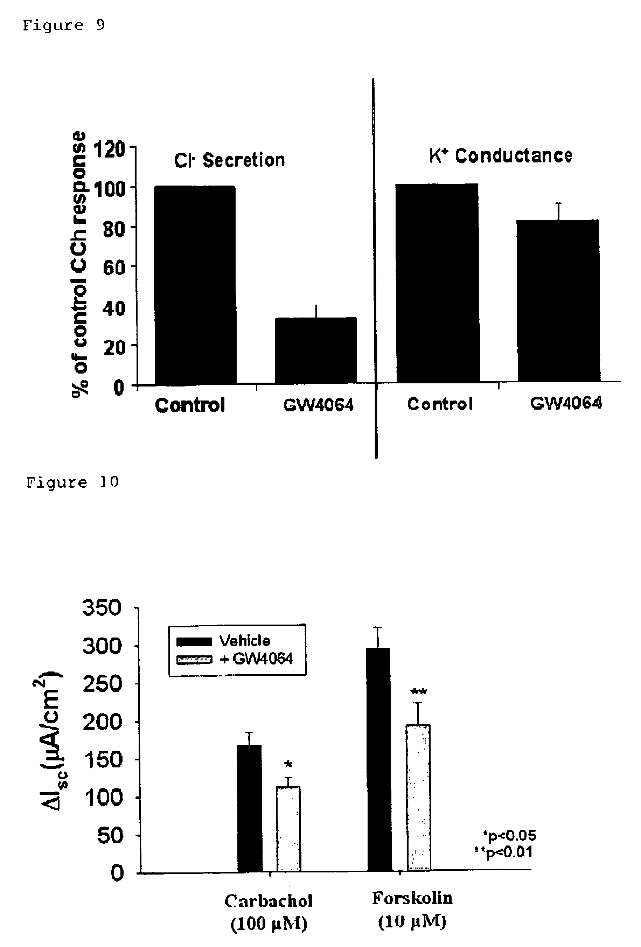

Figure 9: FXR activation does not alter K⁺ channel conductances in colonic epithelial cells: Monolayers of T₈₄ cells were treated with GW4064 (5 µM) for 24 hrs and then mounted in Ussing chambers for measurements of Cl⁻ secretion (left panel) or K⁺ channel conductances (right panel). Although GW4064 inhibited CCh-induced Cl⁻ secretory responses, it did not significantly alter K⁺ conductances (n = 3).
Figure 10: (In-vivo) Administration of a potent and selective FXR agonist inhibits Cl⁻ secretory responses in mouse colon: Mice were injected intraperitoneally with GW4064 (100 mg/kg; 24 hrs) and then sections of muscle-stripped colon were mounted in Ussing chambers for ex vivo I_{sc} measurements, which are known to be predominantly due to Cl⁻ secretion. Pretreatment with GW 4064 significantly attenuated I_{sc} responses to both the Ca²⁺ and cAMP-dependent secretagogues, CCh and forskolin, respectively (n = 8).

### Detailed Description of the Invention

### EXPERIMENTAL (in-vitro)

### Methods:

*Measurements of chloride secretion:* Cultured T₈₄ epithelial cells were employed as a model of the colonic epithelium. T₈₄ cells were grown as monolayers on filter supports. When grown in this way T₈₄ cells retain many of the characteristics of native intestinal epithelium and are widely considered as being among the best reductionist models for analysing intestinal epithelial secretory responses. The T₈₄ cell monolayers were treated in culture with the FXR agonist, GW4064 (1 µM), for 24 hrs. After this time they were washed of GW 4064, mounted in Ussing chambers, and voltage-clamped to zero potential difference. Under these conditions, transepithelial ion transport can be measured as changes in short-circuit current (I_{sc}) and such changes in I_{sc} across T₈₄ cells have been established as being wholly due to Cl⁻ ion secretion. After mounting the cells in Ussing chambers we measured Cl⁻ secretory responses to both the calcium- and cAMP-dependent agonists, carbachol (100µM) and forskolin (10 µM), respectively.

To measure basolateral K⁺ conductance T₈₄ cell monolayers in Ussing chambers were apically permeabilized with amphotericin B (50 µmol/L). A K⁺ gradient (123.2 - 5.2 mmol/L) was created across the basolateral membrane by addition of a high K⁺ (123.2 mmol/L) Ringers solution, in which NaCl is substituted with K⁺-gluconate, to the apical reservoir. Ouabain (100 µmol/L) was added basolaterally to inhibit Na⁺/K⁺-ATPase pump activity. Under these conditions changes in I_{sc} are wholly reflective of changes in basolateral K⁺ conductance (I_{K}⁺).

To measure Na⁺/K⁺-ATPase activity T₈₄ cell monolayers were mounted in Ussing chambers and bathed bilaterally in low-sodium (25 mmol/L) Ringers' solution, where NaCl was substituted with equimolar N-methyl-D-glucamine (NMDG)-Cl⁻ . Apical membranes were permeabilized with amphotericin (50 µmol/L). Under these conditions (ie: in the absence of ionic gradients across the permeabilized monolayer) changes in I_{sc} are wholly reflective of electrogenic transport through the Na⁺/K⁺-ATPase.

*Animal Studies:* Male C57BL6 mice were bred and maintained in an environmentally controlled animal facility at Beaumont Hospital. Experiments were performed on 6-9 week old animals and were in compliance with Irish Department of Health and Children regulations and approved by the Beaumont Hospital Ethics Committee. GW4064 (100 mg/kg), prepared in endotoxin-free PBS, was injected intraperitoneally and mice were sacrificed 24 hours later by cervical dislocation. The distal colon was removed, stripped of underlying smooth muscle, and mounted in Ussing chambers (window area = 0.3 cm²). Results were normalized and expressed as ΔI_{sc} (µA/cm²).

*Western Blotting:* T₈₄ monolayers were washed with ice-cold PBS and lysed (1% Nonidet P-40, 150 mmol/L NaCl, 50 mmol/L Tris Base, 1 x Complete mini EDTA free protease inhibitor tablet, 0.1 mg/1mL PMSF, 1 mmol/L Na₃VO₄). Lysates were collected, centrifuged at 15,294 x g for 10 minutes at 4°C, the pellet discarded, and the supernatant sonicated with 3 x 10 second pulses in order to disrupt membrane proteins. Samples were normalized for protein content and 2x gel loading buffer (50 mM Tris HCl, 100 mM DTT, 40% glycerol and 4% SDS) was added. Samples were then heated at 95°C for 5 min, separated by sodium dodecyl sulphate polyacrylamide gel electrophoresis and transferred to PVDF membranes. Membranes were pre-blocked in 5% blocking buffer (Marvel in PBS) for 60 minutes at room temperature followed by incubation with primary antibody in 5% blocking buffer overnight at 4°C. After washing (x4) in Tris buffered saline with 1% tween (TBST), membranes were incubated with HRP-conjugated secondary antibodies in 5% blocking buffer for 60 min at room temperature. After further washing (x4) in TBST, immunoreactive proteins were detected by enhanced chemiluminescence (Amersham Biosciences, UK).

*Cell Surface Biotinylation:* Following treatment T₈₄ cell monolayers were washed x3 in ice-cold PBS and freshly prepared biotinylation buffer (1 mg/mL sulfo-NHS-biotin (Pierce) in PBS) was added basolaterally. Cells were incubated at 4°C for 15 min on a rotating platform after which the buffer was replaced with a second fresh aliquot. After another 15 min incubation cells were washed, incubated with quenching reagent (100 mmol/L glycine) and lysed for 30 min on ice. The lysate was centrifuged (14,000 x g; 6 min), normalized for protein content, and incubated on a rotator overnight at 4°C with 40µL streptavidin-agarose beads. Samples were then washed (x3) in lysis buffer and 2 x gel loading buffer (50 mmol/L Tris, pH 6.8, 2% SDS, 100 mmol/L dithiothreitol, 0.2% bromophenol blue, 20% glycerol) was added. Samples were heated to 55°C for 30 minutes and subjected to SDS-PAGE. Na⁺/K⁺-ATPase □ subunit was detected by western blot analysis as described above.

*ATP Assay:* T₈₄ cells were cultured on 12mm Millicel Transwells inserts and treated with GW4064 for 24 hours. Cells were then lysed with hypotonic lysis buffer (2 mM EDTA, 100 mM Trizma base, brought to pH 7.75 with acetic acid) and frozen at -80°C. The lysate was later assayed for ATP content using the ENLITEN® ATP Assay System (Promega, Madison, WI).

*Confocal Microscopy:* T₈₄ cells, plated onto Cell-Tack coated chamber slides were fixed in paraformaldehyde and permeabilised in Triton X-100. Samples were incubated with antibodies against FXR, washed and then incubated with fluorescence-conjugated secondary antibodies. Cells were visualised using an LSM 510 META laser scanning confocal microscope.

The data shows that pretreatment of the T₈₄ cell monolayers with GW4064 (1 µM) for 24 hrs significantly attenuates the ability of T₈₄ cells to evoke I_{sc} responses to stimulation with either carbachol or forskolin (Figure 1).

In further experiments the concentration-dependence of GW4064 in exerting its antisecretory actions was examined. Cells were pretreated with GW4064 (0.1 - 10 µM) for 24 hrs after which Cl⁻ secretory responses to both CCh and forskolin were measured (Figure 2). The effects of GW4064 were found to be concentration-dependent with a more marked antisecretory effect being observed against Ca²⁺-dependent secretory responses stimulated by CCh.

Activation of the FXR with GW 4064 significantly inhibits the ability of intestinal epithelial cells to evoke Cl⁻ secretory responses to agonists that act through either the Ca²⁺ or cAMP-dependent signalling pathways. Since Cl⁻ ion secretion is the primary driving force for fluid secretion in the intestine, in vivo, such effects of FXR activation would be expected to inhibit fluid secretion into the intestine. Dysregulated fluid transport, leading to the onset of diarrhoea, is a feature of many intestinal disorders and these data suggest that drugs that target the FXR may be useful in treating such diseases.

GW4064 stimulates FXR translocation to the nucleus. Subcellular localization of FXR (green) following treatment with the FXR agonist, GW4064 was investigated by immunocytochemistry and confocal microscopy. GW4064 (5 µM, 24hr) promoted the translocation of FXR from the cytoplasm to the nucleus (blue). Figure 3

FXR activation attenuates Cl⁻ secretion in a time-dependent fashion: Monolayers of T₈₄ cells were pretreated with GW4064 (5 µM) for various periods of time. Cells were then washed and Cl⁻ secretory responses to CCh (100 µM) were measured in Ussing chambers. Data are expressed as % control I_{sc} responses to CCh (n = 2 - 3). Figure 4.

Effects of FXR activation on T₈₄ cell monolayer conductance: Monolayers of T₈₄ cells were treated with GW4064 at various concentrations for 24 hrs. Monolayers were then mounted in Ussing chambers and transepithelial conductance measurements were made as a measure of cell viability (n = 9 - 13). Only at concentrations > 10 µM did GW 4064 exert toxic effects on T₈₄ cells. Figure 5.

FXR activation inhibits Na⁺/K⁺ ATPase activity in colonic epithelial cells: Monolayers of T₈₄ cells were treated with GW4064 (5 µM) or deoxycholic acid (DCA; 50 µM) for 24 hrs and then mounted in Ussing chambers for measurements of Cl⁻ secretion (left panel; n = 3) or Na⁺/K⁺ ATPase activity (right panel; n = 4). Although both DCA and GW4064 inhibited CCh-induced Cl⁻ secretory responses, only GW 4064 inhibited Na⁺/K⁺ ATPase activity. *** p < 0.001. Figure 6.

FXR activation does not alter abundance or surface expression of the Na⁺/K⁺ ATPase pump in T₈₄ cells: Monolayers of T₈₄ cells were treated with GW4064 (5 µM) for 24 hrs and then cellular abundance of the catalytic α subunit was measured by western blotting (left panel; representative of 3 experiments) or its surface expression was measured by biotinylation (right panel; n = 4). Figure 7.

FXR activation does not alter cellular ATP levels in T₈₄ cells: Monolayers of T₈₄ cells were treated with GW4064 (5 µM) for 24 hrs and then cellular abundance ATP was measured using a commercially available assay kit panel. Data are expressed as % ATP levels in control cells (n = 5). Figure 8.

FXR activation does not alter K⁺ channel conductances in colonic epithelial cells: Monolayers of T₈₄ cells were treated with GW4064 (5 µM) for 24 hrs and then mounted in Ussing chambers for measurements of Cl⁻ secretion (left panel) or K⁺ channel conductances (right panel). Although GW4064 inhibited CCh-induced Cl⁻ secretory responses, it did not significantly alter K⁺ conductances (n = 3). Figure 9.

### EXPERIMENTAL (in-vivo)

Mice were injected intraperitoneally with GW4064 (100 mg/kg; 24 hrs) and then sections of muscle-stripped colon were mounted in Ussing chambers for ex vivo I_{sc} measurements, which are known to be predominantly due to Cl⁻ secretion. Pretreatment with GW 4064 significantly attenuated I_{sc} responses to both the Ca²⁺ and cAMP-dependent secretagogues, CCh and forskolin, respectively (n = 8). Administration of a potent and selective FXR agonist inhibits Cl⁻ secretory responses in mouse colon. Figure 10.

Mice are injected intraperitoneally with INT-747 (100 mg/kg; 24 hrs) and then sections of muscle-stripped colon are mounted in Ussing chambers for ex vivo I_{sc} measurements, which are known to be predominantly due to Cl⁻ secretion. I_{sc} responses to both the Ca²⁺ and cAMP-dependent secretagogues, CCh and forskolin, are determined.

male BALB/c strain mice are sensitized twice, 2 weeks apart, with 50 µg of ovalbumin (OVA) (Sigma-Aldrich, UK) in the presence of 1 mg of aluminum potassium sulfate adjuvant by intraperitoneal injection. A further two weeks later, mice are fasted for 4 hrs and then administered 50 mg of OVA in PBS, or PBS alone, by oral gavage. Mice are challenged with OVA in this way every 2nd day for 2 weeks. Mice are treated with the potent FXR ligand GW-4064 compounds 24 hrs before each challenge with OVA. Diarrhea is assessed by visually monitoring mice for 1 hr following challenge and those demonstrating profuse liquid stool are recorded as being diarrhea-positive.

male BALB/c strain mice are sensitized twice, 2 weeks apart, with 50 µg of ovalbumin (OVA) (Sigma-Aldrich, UK) in the presence of 1 mg of aluminum potassium sulfate adjuvant by intraperitoneal injection. A further two weeks later, mice are fasted for 4 hrs and then administered 50 mg of OVA in PBS, or PBS alone, by oral gavage. Mice are challenged with OVA in this way every 2nd day for 2 weeks. Mice are treated with the potent FXR ligand INT-747 compounds 24 hrs before each challenge with OVA. Diarrhea is assessed by visually monitoring mice for 1 hr following challenge and those demonstrating profuse liquid stool are recorded as being diarrhea-positive.

## Claims

1. Use of a therapeutically effective amount of a potent and selective FXR agonist in the manufacture of a medicament for the symptomatic treatment of diarrhoea.

2. Use as claimed in Claim 1 in which the potent and selective FXR agonist is selected from the group consisting of: 6-ethyl chenodeoxycholic acid (INT-747); a triethyl ammonium salt of 6-ethyl chenodeoxycholic acid; 6-ethyl ursodeoxycholic acid; 3-(2,6-dichlorophenyl)-4-(3'-carbomethoxy-2-chloro-stilben- 4-yl)-oxymethyl-5-isopropyl-isoxazole (GW-4064); and 17-(4-hydroxybenzoyl)androsta-3,5-diene-3-carboxylic acid (MFA-1).

3. Use as claimed in Claim 2 in which the potent and selective FXR agonist is selected from the group consisting of: 6-ethyl chenodeoxycholic acid (INT-747); a triethyl ammonium salt of 6-ethyl chenodeoxycholic acid; 6-ethyl ursodeoxycholic acid; 3-(2,6-dichlorophenyl)-4-(3'-carbomethoxy-2-chloro-stilben- 4-yl)-oxymethyl-5-isopropyl-isoxazole (GW-4064); and 17-(4-hydroxybenzoyl)androsta-3,5-diene-3-carboxylic acid (MFA-1).

4. Use as claimed in Claim 1, 2 or 3 in which the potent FXR agonist has a potency in the nanomolar range.

5. Use as claimed in Claim 4 in which the potent FXR agonist has a potency of from 1 to 500nM.

6. Use as claimed in Claim 4 in which the FXR agonist has a potency of from 1 to 100nM.

7. Use as claimed in Claim 3 in which the potent FXR agonist has a potency of from 10 to 1000 greater than CDCA when determined in a Cell Free Assay.

8. A pharmaceutical composition comprising an anti-microbial agent, a potent and selective FXR agonist, and a suitable pharmaceutical carrier.

9. A pharmaceutical composition of Claim 8 in which the potent and selective FXR agonist is selected from the group consisting of: 6-ethyl chenodeoxycholic acid (INT-747); a triethyl ammonium salt of 6-ethyl chenodeoxycholic acid; 6-ethyl ursodeoxycholic acid; 3-(2,6-dichlorophenyl)-4-(3'-carbomethoxy-2-chloro-stilben- 4-yl)-oxymethyl-5-isopropyl-isoxazole (GW-4064); and 17-(4-hydroxybenzoyl)androsta-3,5-diene-3-carboxylic acid (MFA-1); and potent derivatives thereof.

10. A pharmaceutical composition of Claim 8 in which the potent and selective FXR agonist is selected from the group consisting of: 6-ethyl chenodeoxycholic acid (INT-747); a triethyl ammonium salt of 6-ethyl chenodeoxycholic acid; 6-ethyl ursodeoxycholic acid; 3-(2,6-dichlorophenyl)-4-(3'-carbomethoxy-2-chloro-stilben- 4-yl)-oxymethyl-5-isopropyl-isoxazole (GW-4064); and 17-(4-hydroxybenzoyl)androsta-3,5-diene-3-carboxylic acid (MFA-1).

11. A pharmaceutical composition of Claim 8, 9 or 10 in which the potent FXR agonist has a potency in the nanomolar range.

12. A pharmaceutical composition of any of Claims 8 to 11 in which the potent FXR agonist has a potency of from 1 to 500nM.

13. A pharmaceutical composition of any of Claims 8 to 11 in which the FXR agonist has a potency of from 1 to 100nM.

14. A pharmaceutical composition of any of Claims 8 to 13 in which the potent FXR agonist has a potency of from 10 to 1000 greater than CDCA when determined in a Cell Free Assay.

## Patentansprüche

1. Verwendung einer therapeutisch wirksamen Menge eines wirkstarken und selektiven FXR-Agonisten bei der Herstellung eines Medikaments für die symptomatische Behandlung von Diarrhö.

2. Verwendung nach Anspruch 1, bei der der wirkstarke und selektive FXR-Agonist aus der Gruppe ausgewählt ist, die aus Folgenden besteht: 6-Ethyl-Chenodeoxycholsäure (INT-747); einem Triethylammoniumsalz von 6-Ethyl-Chenodeoxycholsäure, 6-Ethyl-Ursodeoxycholsäure; 3-(2,6-Dichlorphenyl)-4-(3'-carbomethoxy-2-chlorstilben-4-yl)oxymethyl-5-isopropylisoxazol (GW-4064); und 17-(4-Hydroxybenzoyl)-Androsta-3,5-dien-3-carbonsäure (MFA-1).

3. Verwendung nach Anspruch 2, bei der der wirkstarke und selektive FXR-Agonist aus der Gruppe ausgewählt ist, die aus Folgenden besteht: 6-Ethyl-Chenodeoxycholsäure (INT-747); einem Triethylammoniumsalz von 6-Ethyl-Chenodeoxycholsäure; 6-Ethyl-Ursodeoxycholsäure; 3-(2,6-Dichlorphenyl)-4-(3'-carbomethoxy-2-chlorstilben-4-yl)oxymethyl-5-isopropylisoxazol (GW-4064); und 17-(4-Hydroxybenzoyl)-Androsta-3,5-dien-3-carbonsäure (MFA-1).

4. Verwendung nach Anspruch 1, 2 oder 3, bei der der wirkstarke FXR-Agonist eine Wirkstärke im nanomolaren Bereich hat.

5. Verwendung nach Anspruch 4, bei der der wirkstarke FXR-Agonist eine Wirkstärke von 1 bis 500 nM hat.

6. Verwendung nach Anspruch 4, bei der der FXR-Agonist eine Wirkstärke von 1 bis 100 nM hat.

7. Verwendung nach Anspruch 3, bei der der wirkstarke FXR-Agonist eine Wirkstärke des 10- bis 1000-Fachen von CDCA bei Bestimmung in einem zellfreien Assay hat.

8. Pharmazeutische Zusammensetzung, die ein antimikrobielles Mittel, einen wirkstarken und selektiven FXR-Agonisten und einen geeigneten pharmazeutischen Träger umfasst.

9. Pharmazeutische Zusammensetzung nach Anspruch 8, in der der wirkstarke und selektive FXR-Agonist aus der Gruppe ausgewählt ist, die aus Folgenden besteht: 6-Ethyl-Chenodeoxycholsäure (INT-747); einem Triethylammoniumsalz von 6-Ethyl-Chenodeoxycholsäure; 6-Ethyl-Ursodeoxycholsäure; 3-(2,6-Dichlorphenyl)-4-(3'-carbomethoxy-2-chlorstilben-4-yl)oxymethyl-5-isopropylisoxazol (GW-4064); und 17-(4-Hydroxybenzoyl)-Androsta-3,5-dien-3-carbonsäure (MFA-1); und wirkstarken Derivaten davon.

10. Pharmazeutische Zusammensetzung nach Anspruch 8, in der der wirkstarke und selektive FXR-Agonist aus der Gruppe ausgewählt ist, die aus Folgenden besteht: 6-Ethyl-Chenodeoxycholsäure (INT-747); einem Triethylammoniumsalz von 6-Ethyl-Chenodeoxycholsäure; 6-Ethyl-Ursodeoxycholsäure; 3-(2,6-Dichlorphenyl)-4-(3'-carbomethoxy-2-chlorstilben-4-yl)oxymethyl-5-isopropylisoxazol (GW-4064); und 17-(4-Hydroxybenzoyl)-Androsta-3,5-dien-3-carbonsäure (MFA-1).

11. Pharmazeutische Zusammensetzung nach Anspruch 8, 9 oder 10, in der der wirkstarke FXR-Agonist eine Wirkstärke im nanomolaren Bereich hat.

12. Pharmazeutische Zusammensetzung nach einem der Ansprüche 8 bis 11, in der der wirkstarke FXR-Agonist eine Wirkstärke von 1 bis 500 nM hat.

13. Pharmazeutische Zusammensetzung nach einem der Ansprüche 8 bis 11, in der der FXR-Agonist eine Wirkstärke von 1 bis 100 nM hat.

14. Pharmazeutische Zusammensetzung nach einem der Ansprüche 8 bis 13, in der der wirkstarke FXR-Agonist eine Wirkstärke des 10- bis 1000-Fachen von CDCA bei Bestimmung in einem zellfreien Assay hat.

## Revendications

1. Utilisation d'une quantité thérapeutiquement efficace d'un agoniste puissant et sélectif de FXR dans la fabrication d'un médicament pour le traitement symptomatique de la diarrhée.

2. Utilisation selon la revendication 1, dans laquelle l'agoniste puissant et sélectif de FXR est sélectionné dans le groupe consistant en les suivants : acide 6-éthyl-chénodésoxycholique (INT-747) ; un sel d'ammonium triéthylique de l'acide 6-éthyl-chénodésoxycholique ; acide 6-éthyl-ursodésoxycholique ; 3-(2,6-dichlorophényl)-4-(3'-carbométhoxy-2-chloro-stilbén-4-yl)-oxyméthyl-5-isopropyl-isoxazole (GW-4064) ; et acide 17-(4-hydroxybcnzvyi)androsta-3,5-diène-3-carboxylique(MFA-I).

3. Utilisation selon la revendication 2, dans laquelle l'agoniste puissant et sélectif de FXR est sélectionné dans le groupe consistant en les suivants : acide 6-éthyl-chénodésoxycholique (INT-747) ; un sel d'ammonium triéthylique de l'acide 6-éthyl-chénodésoxycholique ; acide 6-éthyl-ursodésoxycholique ; 3-(2,6-dichlorophéryl)-4-(3'-carbométhoxy-2-chloro-stilbén- 4-yl)-oxyméthyl-5-isopropyl-isoxazole (GW-4064) ; et acide 17-(4-hydroxybenzoyl)androsta-3,5-diène-3-carboxylique (MFA-I).

4. Utilisation selon la revendication 1, 2 ou 3, dans laquelle l'agoniste puissant de FXR est actif dans la plage nanomolaire.

5. Utilisation selon la revendication 4, dans laquelle l'agoniste puissant de FXR est actif dans la plage de I-500 nM.

6. Utilisation selon la revendication 4, dans laquelle l'agoniste de FXR est actif dans la plage de I - 100 nM.

7. Utilisation selon la revendication 3, dans laquelle l'agoniste puissant de FXR est 10 à 1 000 fois plus actif que l'acide chénodésoxycholique (CDCA) à la détermination par un test en milieu acellulaire.

8. Composition pharmaceutique comprenant un agent antimicrobien, un agoniste puissant et sélectif de FXR et un véhicule pharmaceutique approprié.

9. Composition pharmaceutique selon la revendication 8, dans laquelle l'agoniste puissant et sélectif de FXR est sélectionné dans le groupe consistant en les suivants : acide 6-éthyl-chénodésoxycholique (INT-747) ; un sel d'ammonium triéthylique de l'acide 6-éthyl-chénodésoxycholique ; acide 6-éthyl-ursodésoxycholique ; 3-(2,6-dichlorophényl)-4-(3'-carbométhoxy-2-chloro-stilbén-4-yl)-oxyméthyl-5-isopropyl-isoxazole (GW-4064) ; et acide 17-(4-hydroxybenzoyl)and rosta-3,5-diène-3-carboxylique (MFA-1) ; et des dérivés actifs de ceux-ci.

10. Composition pharmaceutique selon la revendication 8, dans laquelle l'agoniste puissant et sélectif de FXR est sélectionné dans le groupe consistant en les suivants : acide 6-éthyl-chénodésoxycholique (INT-747) ; un sel d'ammonium triéthylique de l'acide 6-éthyl-chénodésoxycholique ; acide 6-éthyl-ursodésoxycholique ; 3-(2,6-dichlorophényl)-4-(3'-carboniéthoxy-2-chloro-stilbén- 4-yl)-oxyméthyl-5-isopropyl-isoxazole (GW-4064) ; et acide 17-(4-hydroxybenzoyl)androsta-3,5-diène-3-carboxylique(MFA-I).

11. Composition pharmaceutique selon la revendication 8, 9 ou 10, dans laquelle l'agoniste puissant de FXR est actif dans la plage nanomolaire.

12. Composition pharmaceutique selon l'une quelconque des revendications 8 à 11, dans laquel le l'agoniste puissant de FXR est actif dans la plage de 1-500 nM.

13. Composition pharmaceutique selon l'une quelconque des revendications 8 à 11, dans laquelle l'agoniste de FXR est actif dans la plage de 1-100 nM.

14. Composition pharmaceutique selon l'une quelconque des revendications 8 à 13, dans laquelle l'agoniste puissant de FXR est 10 à 1 000 fois plus actif que celle de l'acide chénodésoxycholique (CDCA) à la détermination par un test en milieu acellulaire.
